(19) Europäisches Patentamt / European Patent Office / Office européen des brevets

(11) **EP 2 054 028 B1**

(12) **EUROPEAN PATENT SPECIFICATION**

(45) Date of publication and mention of the grant of the patent:
**27.06.2012 Bulletin 2012/26**

(21) Application number: **07788130.8**

(22) Date of filing: **01.08.2007**

(51) Int Cl.:
**A61K 9/00** (2006.01)

(86) International application number:
**PCT/EP2007/057961**

(87) International publication number:
**WO 2008/015232 (07.02.2008 Gazette 2008/06)**

(54) **SUBCUTANEOUS IMPLANTS RELEASING AN ACTIVE PRINCIPLE OVER AN EXTENDED PERIOD OF TIME**

SUBKUTANE IMPLANTATE, DIE ÜBER EINEN LÄNGEREN ZEITRAUM EINEN WIRKSTOFF FREISETZEN

IMPLANTS SOUS-CUTANÉS LIBÉRANT UN PRINCIPE ACTIF PENDANT UNE DURÉE ÉTENDUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.08.2006 IT MI20061539**

(43) Date of publication of application:
**06.05.2009 Bulletin 2009/19**

(73) Proprietor: **Mediolanum Pharmaceuticals Limited Dublin 4 (IE)**

(72) Inventors:
• **MAURIAC, Patrice**
  **F-75012 Paris (FR)**
• **MARION, Pierre**
  **F-93360 Neuilly Plaisance (FR)**

(74) Representative: **Cattaneo, Elisabetta**
**Notarbartolo & Gervasi S.p.A**
**Corso di Porta Vittoria, 9**
**20122 Milano (IT)**

(56) References cited:
**WO-A-2005/000277       WO-A-2005/000278**
**US-A1- 2005 244 472**

• **RENU BHARDWAJ; ET AL.: "In vitro evaluation or Poly(D,L-lactide-co-glycolide) polymer-based implants containing the alpha-melanocyte stimulating hormone Melanotan-I" JOURNAL OF CONTROLLED RELEASE, vol. 45, no. 1, 1997, pages 49-55, XP004099354**

## Description

### Field of the invention

The present invention relates to subcutaneous implants obtained by extrusion containing an active ingredient, and a hydrophilic excipient dispersed in a PLGA matrix.

### State of the art

Many active ingredients are rapidly metabolized and eliminated by the human or mammalian organism, therefore requiring frequent administration of the drug with the aim of maintaining an adequate therapeutic concentration.

An example of controlled release implants are represented by subcutaneous implants.

Among the numerous implants previously described, the subcutaneous implants described in WO00/33809 represent a net improvement with reference to previous subcutaneous implants containing as the active principle a polypeptide dispersed in a matrix of polylactic-glycolic acid in that they are able to release the aforesaid active principle in 6 months. The subcutaneous implants described in said previous patent differ also in that they present an essentially triphasic and not biphasic release profile as clarified in the following manner: release by pure diffusion, diffusion by release following to swelling and release by polymer degradation.

This progression therefore allows for an extension of release times. In fact when these implants are introduced into an aqueous medium, the water diffuses through the polymeric matrix reaching the peptide particles closest to the surface and subsequently the inner zones.

The implant remains substantially unmodified for about 6 weeks and in this period releases approximately 30% of the peptide.

The duration of this stage of pure diffusion is essentially determined by the level of heterogeneity of the peptide dimensions and the rate is essentially determined by the particle content in the PLGA matrix.

As the active principle presents heterogeneous dimensions, a sufficient quantity of peptide remains after the first stage of dissolution and can be released in the successive stages mentioned, that is release by diffusion and swelling, or release by disintegration of the polymer.

Subcutaneous implants including those above described suffer from a drawback essentially caused by the fact that the active ingredient release rate during the three successive phases is partially governed by the concentration of active ingredient within the polymeric matrix (the other factors being the intrinsic solubility and diffusion properties of the active ingredient and the characteristics of the PLGA). On one hand, the amount of active ingredients (dose) to be incorporated into the implant depends on the activity of the product and on the expected dosing interval. However big dimensions implants create problem insofar patients compliance is concerned on the other hand small dimensions subcutaneous implants create problems under industrial feasibility aspects since a very thin or very short implant may be difficult to handle and to package.

It may therefore happen that implants containing a small amount of very potent active ingredient within a "normal" size (i.e. around 1 mm diameter and 1 cm length) presents a very low active ingredient concentration within the polymeric matrix, thus leading to a poor release profile especially in the first two weeks from the administration, afterwards a marked and abrupt release resulting in an overall shortened period if compared to subcutaneous implants containing higher amounts of the active ingredient.

Subctaneous implants (Renu Bhardwaj et al. "In vitro evaluation or poly(D,L-lactide-co-glycolide) polymer-based implants containing the alpha-melanocyte stimulating hormone Melatonan-I", Journal of controlled release, vol. 45, no.1, 1997, pages 49-55) containing the active principle Melaton-I in the range from 2 to 10%wt have been described. Specifically such a document discloses the incorporation of sodium chloride or methylcellulose with an increase of the peptide release rate during the secondary phase of the release pattern. However, such implants do not guarantee a three-months release with an more gradual release overall with also an increase release in the first phase.

### Summary of the invention

The Applicant has now unexpectedly found subcutaneous PLGA based implants formulations which overcome the above drawback.

The present invention therefore relates to subcutaneous implants composed of a PLGA based polymeric matrix containing dispersed therein an active ingredient and a hydrophilic excipient so that the weight ratio:

(Active Ingredient (AI) + Excipient (E))/PLGA is higher than 0.05 and lower than 1,

and wherein the active principle is chosen from the class consisting of a peptide, an analgesic-narcotic active ingredient and has a particle size distribution comprised between 1 and 63$\mu$m and the hydrophilic excipient is selected from mannitol, sorbitol, trehalose, polyvinylpyrrolidone having an average molecular weight of from 6000 to 10000 Da dispersed in a matrix consisting of PLGA and

wherein when the hydrophilic excipient is mannitol it is present in weight ratio with respect to the active ingredient ranging from 2:1 to 5:1.

In fact the subcutaneous having said weight ratio lower than 0.05 behave like the aforesaid subcutaneous implants containing a low amount of active ingredient in the absence of hydrophilic excipient, namely they exhibit a poor release profile especially in the first two weeks from the administration, afterwards a marked and abrupt release resulting in an overall shortened period if compared to subcutaneous implants containing higher amounts of the active ingredient, whereas the subcutaneous implants having the aforesaid weight ratio equal to or higher than 1, release too quickly the active ingredient.

On the contrary the implants of the invention result to release the active ingredient therein contained with the typical triphasic profile and the presence of this excipient within the formulation modify the release rates during the three successive phases and also modify the overall release duration.

## Description of the figure

Figure 1 shows, in ordinates, the active ingredient release (% of the total amount released) versus, in abscissa, time expressed in days after immersion in the aqueous medium of the implants described of Example 1.

Figure 2 shows, in ordinates, the active ingredient release (% of the dose) versus, in abscissa, time expressed in days after immersion of the implants described of Example 2.

Figure 3 shows, in ordinates, the active ingredient release (mg of active ingredient) versus, in abscissa, time expressed in days after immersion of the formulations 2#1 and 2#2.

Figure 4 shows, in ordinates, the active ingredient release (% of the total amount released) versus, in abscissa, time expressed in days after immersion of the implants described of Example 3.

## Detailed description of the invention

The aforesaid weight ratio is preferably comprised between 0.3 and 0.9, more preferably between 0.4 and 0.8.

The hydrophilic excipient is preferably selected from mannitol, sorbitol, trehalose, polyvinylpyrrolidone having an average molecular weight ranging from 6000 to 10000 Da more preferably 8000 Da.

The subcutaneous implants of the present invention preferably contain an active principle chosen from the class consisting of: a peptide, an analgesic-narcotic active principle.

More preferably said peptide is chosen from: avorelin, triptorelin, goserelin and leuprorelin.

As "drugs with narcotic analgesic activity" preferred are morphine and morphinans, i.e. compounds having a chemical structure and activity similar to that of morphine i.e. $\mu$ receptor agonists, but also compounds with morphinic-type activity, in other words also $\mu$ receptor agonists but with a different chemical structure such as those belonging to the phenylpiperidine class. (Goodman & Gilman's "The pharmacological basis of therapeutics "Ninth Edition Chapter 23 pages 521-555). As phenylpiperidine $\mu$ receptor agonists we cite as preferred at least one active principle chosen from the class consisting of meperidine, fentanyl and relative pharmaceutically acceptable salts, fentanyl congeners, for example sufentanyl, alfentanyl, lofentanyl, carfentanyl, remifentanyl and their pharmaceutically acceptable salts.

Preferably, when the subcutaneous implants according to the present invention contain a peptide as the active ingredient, they show a heterogeneous particles size distribution more preferably ranging from 1 to 63$\mu$m or from 1 to 100 $\mu$m. Specifically, when the subcutaneous implants of the invention contain the peptides having the aforesaid heterogeneous particles size dimensions, also the hydrophilic excipient has heterogeneous particles size distribution preferably ranging from 10 to 250 $\mu$m.

When the hydrophilic excipient is mannitol it is preferably present in the subcutaneous implants in weight ratio with respect to the active ingredient in amounts ranging from 2:1 to 5:1 and more preferably in weight ratio of 4:1.

When the hydrophilic excipient is trehalose or polyvinylpyrrolidone it is preferably present in weight ratio with respect to the active ingredient in amounts ranging from 1:6 to 1:1, more preferably of from 1: 5 to 1:2.

The PLGA contained in the subcutaneous implants according to the present invention has preferably a weight average molecular weight of from 50000.to 150000 Da and a lactic acid/ glycolic acid ranging from 50/50 to 95/5.

As PLGA polymeric matrix the subcutaneous implants according to the present invention may contain a sole PLGA or that obtained by grinding an extruded product of a blend of:

■ at least two PLGA having different lactic acid/ glycolic acid molar ratios and different weight average molecular weights,
■ a PLGA and PLA having different weight average molecular weights subject matter of the co-pending application filed in the same day of the instants Patent Application.

The present invention further relates to

☐ the process of preparation of the subcutaneous implants containing a sole PLGA which comprises the following steps:

a) dry- mixing the active ingredient and the hydrophilic excipient,

b) dry mixing or wet granulating the mixture obtained in step (a) with PLGA in a suitable solvent

c) drying the wet granulated mixture coming from step (b) up to a maximum solvent content of from 0.5 to 3%

d) extruding the dried granulated mixture coming from step (c) or the dry mixture coming from step (b).

☐ the process of preparation of the subcutaneous implants according to the present invention containing a PLGA obtained by grinding an extruded product of a blend of:

• - at least two PLGA having different lactic acid/ glycolic acid molar ratios and different weight average molecular weights,
• - a PLGA and PLA having different weight average molecular weights, which comprises the following steps:

A) Mixing at least two PLGA having different weight average molecular weight and different lactic acid/ glycolic acid molar ratio or PLGA with PLA having different weight average molecular weights,
B) extruding the powder mixture coming from step (a) and then grinding the extruded PLGA mixture, thereby obtaining granules of the blended extruded PLGA,
C) dry- mixing the active ingredient and the hydrophilic excipient,
D) dry mixing or (D') wet granulating in a suitable solvent the mixture obtained in step (B) with PLGA coming from step (C)
E) drying the wet granulated mixture coming from step (D') up to a maximum solvent content of from 0.5 to 3%
F) extruding the dried granulated mixture coming from step (E) or the dry mixture coming from step (D).

We report herewith for illustrative but not limiting purposes an example of preparation of subcutaneous implants according to the present invention.

EXAMPLE 1- preparation of subcutaneous implants containing Avorelin (formulations No. Med 011, Med 012 and Med 013)

Subcutaneous implants containing ingredients as described in the table below are prepared as described in WO00/33809

| Form. Nr | Avorelin (having particle size distribution ranging from 1 to 63 $\mu$m) | PLGA (UG molar ratio 54/46 -molecular weight 51 kg/mol) | Mannitol (excipient) | Excipient VS Active Ingredient ratio (E/AI) | Active ingredient + Excipient VS PLGA ratio (AI+E/ PLGA) |
|---|---|---|---|---|---|
| 1#1 | 25% w/w | 75% w/w | 0% w/w | NA | 1/3 |
| 1#2 | 5% w/w | 75% w/w | 20% w/w | 4/1 | 1/3 |
| 1#3 | 5% w/w | 95% w/w | 0% w/w | NA | 1/19 |

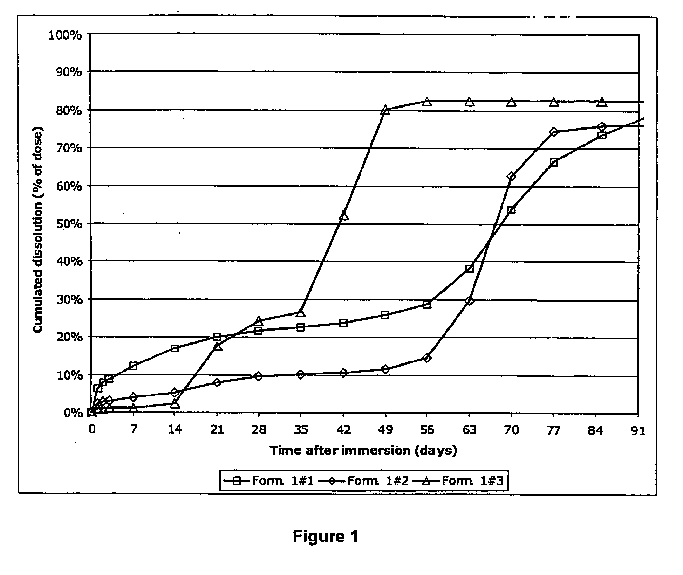

Figure 1 shows, in ordinates, the active ingredient release (% of the total amount released) versus, in abscissa, time expressed in days after immersion of the implants described of Example 1.

It is observed that the typical triphasic release pattern and a 3 months release duration are obtained with 25.0 % w/w loading of active agent (Form. 1#1). On the contrary neither the profile nor the duration is maintained when loading the matrix at 5.0% w/w (Form. 1#3). Finally the triphasic profile and the 3 months duration are recovered when adding 20.0% w/w of mannitol to the 5.0% w/w of active agent (1#2).

In the case of the low active ingredient loading ((AI+E)/PLGA ratio = 1/19), a very limited number of channels exists within the polymeric matrix. The degradation of the polymeric matrix through autocatalysis is therefore accelerated (this leading to a shorter overall release duration). When adding 20% of a very hydrophilic small molecule (Mannitol), a lot of channels are opened so that the circulation of dissolution buffer within the matrix is sufficient to limit the autocatalysis process and, as a consequence, the release of active ingredient are higher in the first weeks than with subcutaneous implants containing the same amount of active ingredient in the sole PLGA and contemporaneously the matrix resists for longer time to hydrolysis.

**EXAMPLE 2- preparation of subcutaneous implants containing Avorelin**

Subcutaneous implants containing ingredients as described in the table below are prepared as described in WO00/33809

| Form. Nr | Avorelin (having particle size distribution ranging from 1 to 63 $\mu$m) | PLGA (UG molar ratio 50/50 -molecular weight 100 kg/mol) | Trehalose (excipient) | Excipient VS Active ingredient ratio (E/AI) | Active ingredient + Excipient VS PLGA ratio (AI+E/ PLGA) |
|---|---|---|---|---|---|
| 2#1 | 29% w/w | 71 % w/w | 0% w/w | NA | ≈ 2/5 |
| 2#2 | 20% w/w | 71 % w/w | 9% w/w | ≈ 1/2 | ≈ 2/5 |
| 2#3 | 20% w/w | 49% w/w | 31 % w/w | 3/2 | ≈ 1/1 |

A 40 mg implant according to formulation 2#1 contains 11.6 mg of Active ingredient when, according to formulations 2#2 and 2#3, the same implant contains 8 mg of active ingredient.

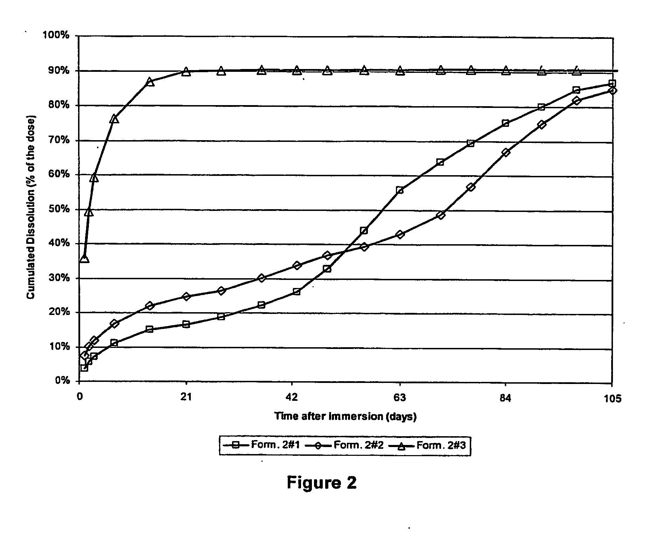

Figure 2 shows, in ordinates, the active ingredient release (% of the dose) versus, in abscissa, time expressed in days after immersion of the implants described of Example 2.

It is observed that both formulations 2#1 and 2#2 present the typical triphasic release pattern and a 3 months long release duration. With a similar (≈ 2/5) AI+E/PLGA ratio, these two formulations operate properly even if it is also noticed in this case that the presence of a small hydrophilic molecule (trehalose) tends to increase the dissolution rate during the first month and to delay the second burst (PLGA degradation driven).

The dissolution profile from formulation 2#3 is also very informative. In this case, AI+E/PLGA ratio is close to 1/1. This means that half of the matrix is occupied by very hydrophilic molecules. Once dropped into the dissolution medium, such an implant presents a huge number of channels allowing the active ingredient to leave the matrix (through a percolation process).

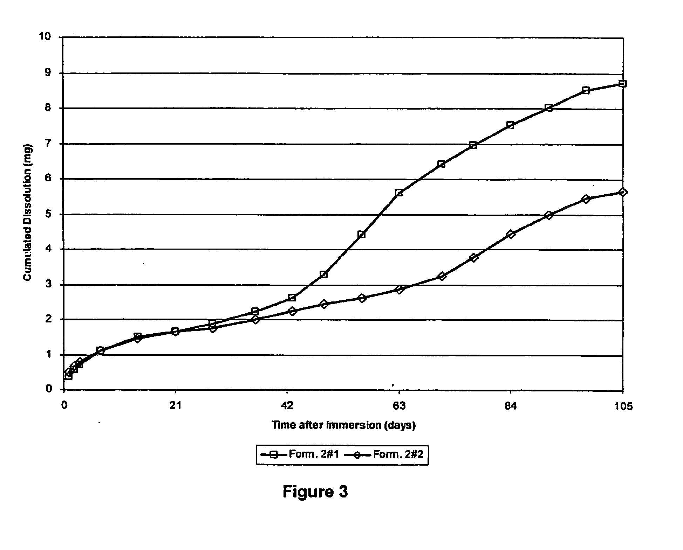

Figure 3 shows, in ordinates, the active ingredient release (mg of active ingredient) versus, in abscissa, time expressed in days after immersion of the formulations 2#1 and 2#2.

It is interesting to notice that formulation 2#1 (containing 11.6 mg of active ingredient per depot) and formulation 2#2 (containing 8.0 mg of active ingredient per depot) release almost the same amount of active ingredient over the entire first month after immersion.

The use of an excipient is clearly a powerful formulation tool. It is useful to allow for a specific implant to operate properly even with low active ingredient loading but it is also useful to modulate a suboptimal release profile up to exactly reach the target

EXAMPLE 3- preparation of subcutaneous implants containing Fentanyl citrate

Subcutaneous implants containing ingredients as described in the table below are prepared as described in WO00/33809

| Form. Nr | Fentanyl citrate (having particle size distribution ranging from 1 to 63 μm) | PLGA (UG molar ratio 75/25 -molecular weight 120 kg/mol) | Excipient | Excipient VS Active ingredient ratio (E/AI) | Active ingredient + Excipient VS PLGA ratio (AI+E/PLGA) |
|---|---|---|---|---|---|
| 3#1 | 36% w/w | 64% w/w | 0% w/w | NA | ≈ 3/5 |
| 3#2 | 36% w/w | 57% w/w | 7% w/w PVP 8KDa | ≈ 1/5 | ≈¾ |
| 3#3 | 36% w/w | 57% w/w | 7% w/w Mannitol | ≈ 1/5 | ≈¾ |

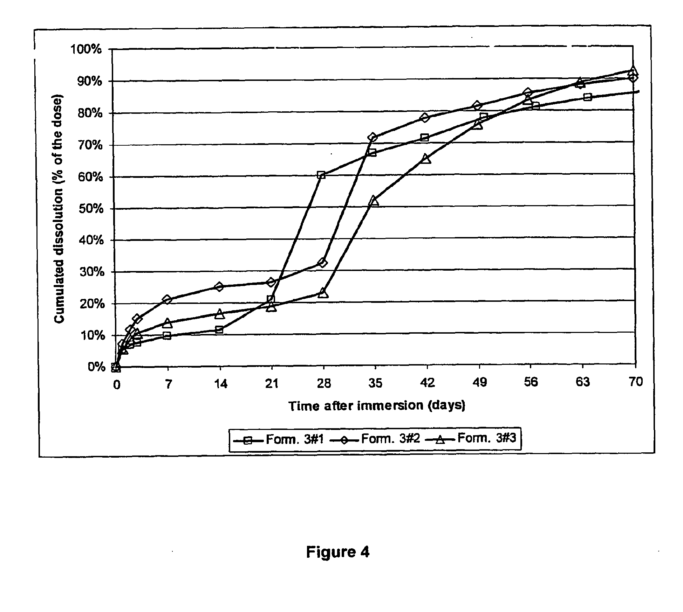

Figure 4 shows, in ordinates, the active ingredient release (% of the total amount released) versus, in abscissa, time expressed in days after immersion of the implants described of Example 3.

Figure 4 demonstrates that, also with a small hydrophilic active molecule, adding an hydrophilic inactive substance results in increasing the initial release and delaying the PLGA degradation process.

It is also observed that, in this case, polyvinyl pyrrolidone (PVP) appears to show better hydrophilicity if compared to Mannitol added at the same weight as PVP

**Claims**

1. Subcutaneous implants obtained by extrusion containing an active ingredient, chosen from the class consisting of: a peptide, an analgesic-narcotic active ingredient and having a particle size distribution comprised between 1 and 63μm, and a hydrophilic excipient selected from mannitol and sorbitol, trehalose, polyvinyl pyrrolidone having an average molecular weight of from 6000 to 10000 Da dispersed in a matrix consisting of PLGA, so that the weight ratio: (Active Ingredient (AI) + Excipient (E))IPLGA is higher than 0.05 and lower than 1 and wherein when the hydrophilic excipient is mannitol it is present in weight ratio with respect to the active ingredient ranging from 2:1 to 5:1.

2. Subcutaneous implants according to claim 1 wherein said weight ratio is comprised between 0.3 and 0.9.

3. The subcutaneous implants according to claim 1 wherein said weight ratio is comprised between 0.4 and 0.8

4. Subcutaneous implants according to claim 1 wherein the average molecular weight of polyvinylpyrrolidone is 8000 Da.

5. Subcutaneous implants according to claim 1 , wherein said peptide is selected from the group consisting of: avorelin, triptorelin, goserelin and leuprorelin.

6. Subcutaneous implants according to claim 1 the active ingredient with narcotic analgesic activity are morphine and morphinans, μ receptor agonists, and compounds with morphinic-type activity of phenylpiperidine class.

7. Subcutaneous implants according to claim 6 wherein the phenylpiperidine μ receptor agonists are chosen from the class consisting of meperidine, fentanyl fentanyl congeners and relative pharmaceutically acceptable salts thereof.

8. Subcutaneous implants according to claim 1 , wherein also the hydrophilic excipient has heterogeneous particles size distribution ranging from 10 to 250 μm.

9. Subcutaneous implants according to claim 1 wherein said weight ratio is 4:1.

10. Subcutaneous implants according to claim 1 wherein when the hydrophilic excipient is selected from trehalose or polyvinlypyrrolidone it is present in weight ratio with respect to the active ingredient in amounts ranging from 1:6 to 1:1.

11. The subcutaneous implants according to claim 10, wherein said weight ratio is comprised between 1:5 to 1:2.

12. Subcutaneous implants according to anyone of claims 1-11, wherein the PLGA contained in the subcutaneous implants according to the present invention has a weight average molecular weight of from 50000.to 150000 Da and a lactic acid/ glycolic acid ranging from 50/50 to 95/5.

13. Subcutaneous implants according to anyone of claims 1-12, containing a sole PLGA or that obtained by grinding an extruded product of a blend of:

   • at least two PLGA having different lactic acid/ glycholic acid molar ratios and different weight average molecular weights,
   • a PLGA and PLA having different weight average molecular weights

14. A process for preparing the subcutaneous implants according to claim 13 containing a sole PLGA which comprises the following steps:

   (a) dry- mixing the active ingredient and the hydrophilic excipient,
   (b) dry mixing or (b') wet granulating the mixture obtained in step (a) with PLGA in a suitable solvent
   (c) drying the wet granulated mixture coming from step (b) up to a maximum solvent content of from 0.5 to 3%
   (d) extruding the dried granulated mixture coming from step (c) or the dry mixture coming from step (b).

15. A process for preparing the subcutaneous implants according to claim 13, which comprises the following steps:

   A) Mixing at least two PLGA having different weight average molecular weight and different lactic acid/ glycolic acid molar ratio, or the PLGA with PLA having different weight average molecular weight,
   B) extruding the powder mix coming from step (A) and then grinding the extruded PLGA mixture, thereby obtaining granules of the blended extruded PLGA,
   C) dry- mixing the active ingredient and the hydrophilic excipient,
   D) dry mixing or wet granulating in a suitable solvent the mixture obtained in step (B) with PLGA coming from step (C)
   E) drying the wet granulated mixture coming from step (D) up to a maximum solvent content of from 0.5 to 3%
   F) extruding the dried granulated mixture coming from step (E) or the dry mixture coming from step (D).

**Patentansprüche**

1. Subkutane Implantate erhalten durch Extrusion, enthaltend einen Wirkstoff, ausgewählt aus der Klasse, bestehend aus: einem Peptid, einem narkotisch-analgetischen Wirkstoff, und mit einer Verteilung der Partikelgröße, umfassend zwischen 1 und 63 $\mu$m, sowie einen hydrophilen Trägerstoff, ausgewählt aus Mannitol und Sorbitol, Trehalose, Polyvinylpyrrolidon mit einem durchschnittlichen Molekulargewicht von 6000 bis 10000 Da, dispergiert in einer Matrix, bestehend aus PLGA, sodass das Gewichtsverhältnis:
(Wirkstoff (W) + Trägerstoff (T)/PLGA höher als 0,05 und niedriger als 1 ist, und wobei wenn der hydrophile Trägerstoff Mannitol ist, dieser in einem Gewichtsverhältnis vorliegt, welches in Bezug zu dem Wirkstoff im Bereich von 2:1 bis 5:1 liegt.

2. Subkutane Implantate gemäß Anspruch 1, wobei genanntes Gewichtsverhältnis zwischen 0,3 und 0,9 umfasst.

3. Subkutane Implantate gemäß Anspruch 1, wobei genanntes Gewichtsverhältnis zwischen 0,4 und 0,8 umfasst.

4. Subkutane Implantate gemäß Anspruch 1, wobei das durchschnittliche Molekulargewicht von Polyvinylpyrrolidon 8000 Da beträgt.

5. Subkutane Implantate gemäß Anspruch 1, wobei genanntes Peptid aus der Gruppe ausgewählt ist, bestehend aus: Avorelin, Triptorelin, Goserelin und Leuprorelin.

6. Subkutane Implantate gemäß Anspruch 1, wobei es sich bei dem Wirkstoff mit narkotisch-analgetischer Wirkung um Morphin und Morphinane, $\mu$-Rezeptor-Agonisten und Verbindungen der Phenylpiperidin-Klasse mit morphinähnlicher Wirkung handelt.

7. Subkutane Implantate gemäß Anspruch 6, wobei die Phenylpiperidin-$\mu$-Rezeptor-Agonisten aus der Klasse ausgewählt sind, bestehend aus Meperidin, Fentanyl-Kongeneren und entsprechenden pharmazeutisch anerkannten Salzen davon.

8. Subkutane Implantate gemäß Anspruch 1, wobei auch der hydrophile Trägerstoff heterogene Partikelgrößenverteilung im Bereich von 10 bis 250 μm aufweist.

9. Subkutane Implantate gemäß Anspruch 1, wobei genanntes Gewichtsverhältnis 4:1 beträgt.

10. Subkutane Implantate gemäß Anspruch 1, wobei wenn der hydrophile Trägerstoff aus Trehalose oder Polyvinylpyrrolidon ausgewählt ist, dieser in einem Gewichtsverhältnis vorliegt, das in Bezug auf den Wirkstoff im Bereich von 1:6 bis 1:1 liegt.

11. Subkutane Implantate gemäß Anspruch 10, wobei genanntes Gewichtsverhältnis Bereiche zwischen 1:5 bis 1:2 umfasst.

12. Subkutane Implantate gemäß einem der Ansprüche 1-11, wobei das in den subkutanen Implantaten enthaltene PLGA gemäß der vorliegenden Erfindung eine gewichtsdurchschnittliche Molmasse von 50000 bis 150000 Da sowie ein Verhältnis von Milchsäure/Glycolsäure im Bereich von 50/50 bis 95/5 aufweist.

13. Subkutane Implantate gemäß einem der Ansprüche 1-12, enthaltend ein einziges PLGA oder das durch Vermahlen erhaltene extrudierte Produkt einer Mischung aus:

• mindestens zwei PLGA mit unterschiedlichen molaren Verhältnissen von Milchsäure/Glycolsäure und unterschiedlichen gewichtsdurchschnittlichen Molmassen,
• einem PLGA und PLA mit unterschiedlichen gewichtsdurchschnittlichen Molmassen.

14. Verfahren zur Herstellung der subkutanen Implantate gemäß Anspruch 13, enthaltend ein einziges PLGA, welches die folgenden Schritte umfasst:

(a) Trockenmischen des Wirkstoffs und des hydrophilen Trägerstoffs;
(b) Trockenmischen oder (b') Feuchtgranulieren der in Schritt (a) erhaltenen Mischung mit PLGA in einem geeigneten Lösungsmittel;
(c) Trocknen der feuchtgranulierten aus Schritt (b) stammenden Mischung bis zu einem maximalen Lösungsmittelgehalt von 0,5 bis 3 %;
(d) Extrudieren der getrockneten granulierten aus Schritt (c) stammenden Mischung oder der aus Schritt (b) stammenden trockenen Mischung.

15. Verfahren zur Herstellung der subkutanen Implantate gemäß Anspruch 13, welches die folgenden Schritte umfasst:

A) Mischen von mindestens zwei PLGA mit unterschiedlichen gewichtsdurchschnittlichen Molmassen und unterschiedlichem molarem Verhältnis von Milchsäure/Glycolsäure oder von dem PLGA mit PLA mit unterschiedlicher gewichtsdurchschnittlicher Molmasse;
B) Extrudieren der aus Schritt (A) stammenden Pulvermischung und anschließendem Zermahlen der extrudierten PLGA-Mischung, wodurch Granulate des gemischten extrudierten PLGA erhalten werden,
C) Trockenmischen des Wirkstoffs und des hydrophilen Trägerstoffs,
D) Trockenmischen oder Feuchtgranulieren der in Schritt (B) erhaltenen Mischung in einem geeigneten Lösungsmittel mit PLGA aus Schritt (C),
E) Trockenen der feuchtgranulierten aus Schritt (D) stammenden Mischung bis zu einem maximalen Lösungsmittelgehalt von 0,5 bis 3 %,
F) Extrudieren der getrockneten granulierten aus Schritt (E) stammenden Mischung oder der trockenen Mischung aus Schritt (D).

**Revendications**

1. Implants sous-cutanés obtenus par extrusion contenant un ingrédient actif, sélectionné dans la classe consistant en: un peptide, un ingrédient actif analgésique-narcotique et ayant une distribution de taille de particule comprise entre 1 et 63μm, et un excipient hydrophile sélectionné parmi mannitol et sorbitol, tréhalose, polyvinyl pyrrolidone d'un poids moléculaire moyen de 6000 à 10 000 Da dispersé dans une matrice consistant en PLGA de sorte que le rapport pondéral: (Ingrédient Actif (AI) + Excipient (E)/PLGA est plus élevé que 0,05 et plus bas que 1, et où, lorsque l'excipient hydrophile est le mannitol, il est présent en un rapport pondéral relativement à l'ingrédient actif allant de 2:1 à 5:1.

**2.** Implants sous-cutanés selon la revendication 1, où le rapport pondéral est compris entre 0,3 et 0,9.

**3.** Implants sous-cutanés selon la revendication 1, où le rapport pondéral est compris entre 0,4 et 0,8.

**4.** Implants sous-cutanés selon la revendication 1, où le poids moléculaire moyen du polyvinylpyrrolidone est de 8000 Da.

**5.** Implants sous-cutanés selon la revendication 1, où ledit peptide est sélectionné dans le groupe consistant en: avoréline, triptolérine, goséréline et leuproréline.

**6.** Implants sous-cutanés selon la revendication 1, l'ingrédient actif avec l'activité narcotique analgésique sont la morphine et les morphinanes, des antagonistes du récepteur μ et des composés avec une activité de type morphinique de la classe de phénylpipéridine.

**7.** Implants sous-cutanés selon la revendication 6, où les antagonistes du récepteur de la phénylpipéridine μ sont sélectionnées dans la classe consistant en mépéridine, fentanyl, congénères de fentanyl et des sels relatifs pharmaceutiquement acceptables de ceux-ci.

**8.** Implants sous-cutanés selon la revendication 1, où également l'excipient hydrophile a une distribution de la taille de particule hétérogène allant de 10 à 250 μm.

**9.** Implants sous-cutanés selon la revendication 1, où le rapport pondéral est de 4:1.

**10.** Implants sous-cutanés selon la revendication 1, où lorsque l'excipient hydrophile est sélectionné parmi tréhalose ou polyvinylpyrrolidone, il est présent en un rapport pondéral relativement à l'ingrédient actif dans des quantités allant de 1:6 à 1:1.

**11.** Implants sous-cutanés selon la revendication 10, où ledit rapport pondéral est compris entre 1:5 à 1:2.

**12.** Implants sous-cutanés selon l'une quelconque des revendications 1 à 11, où le PLGA se trouvant dans les implants sous-cutanés selon la présente invention a une masse moléculaire moyenne en poids de 50 000 à 150 000 Da et un acide lactique/acide glycolique de 50/50 à 95/5.

**13.** Implants sous-cutanés selon l'une quelconque des revendications 1 à 12, contenant un PLGA seul ou celui obtenu par le meulage d'un produit extrudé d'un mélange de:

• au moins deux PLGA ayant des rapports molaires d'acide lactique/d'acide glycolique différents et différentes masses moléculaires moyennes en poids,
• un PLGA et un PLA ayant des masses moléculaires moyennes en poids différentes.

**14.** Procédé de préparation des implants sous-cutanés selon la revendication 13 contenant un seul PLGA qui comprend les étapes suivantes:

(a) mélanger à sec l'ingrédient actif et l'excipient hydrophile,
(b) mélanger à sec ou (b') granuler à l'état humide le mélange obtenu à l'étape (a) avec du PLGA dans un solvant approprié,
(c) sécher le mélange granulé humide provenant de l'étape (b) jusqu'à une teneur en solvant maximum de 0,5 à 3%,
(d) extruder le mélange granulé séché provenant de l'étape (c) ou le mélange sec provenant de l'étape (b).

**15.** Procédé de préparation des implants sous-cutanés selon la revendication 13, qui comprend les étapes suivantes:

A) mélanger au moins deux PLGA ayant des masses moléculaires moyennes en poids différentes et un rapport molaire d'acide lactique/d'acide glycolique différent, ou bien le PLGA avec le PLA ayant une masse moléculaire moyenne en poids différente,
B) extruder le mélange de poudre provenant de l'étape (A) et meuler ensuite le mélange PLGA extrudé en obtenant ainsi des granules du PLGA mélangé extrudé,
C) mélanger à sec l'ingrédient actif et l'excipient hydrophile,

D) mélanger à sec ou granuler à l'état humide dans un solvant approprié le mélange obtenu à l'étape (B) avec le PLGA provenant de l'étape (C)

E) sécher le mélange granulé humide provenant de l'étape (D) jusqu'à une teneur maximum en solvant de 0,5 à 3%,

F) extruder le mélange granulé séché provenant de l'étape (E) ou le mélange sec provenant de l'étape (D).

**Figure 1**

**Figure 2**

**Figure 3**

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• WO 0033809 A

### Non-patent literature cited in the description

• **RENU BHARDWAJ et al.** In vitro evaluation or poly(D,L-lactide-co-glycolide) polymer-based implants containing the alpha-melanocyte stimulating hormone Melatonan-I. *Journal of controlled release, vol.,* 1997, vol. 45 (1), 49-55

• **GOODMAN ; GILMAN'S.** The pharmacological basis of therapeutics. vol. 23, 521-555